# EUROPEAN PATENT APPLICATION

(11) **EP 1 172 443 A1**
(43) Date of publication of application: **16.01.2002**
(21) Application number: 00909724.7
(22) Date of filing: 17.03.2000
(51) Int. Cl.: C12Q 1/37, C12N 9/99, C07D 497/20, C07D 497/22, A61K 31/438, A61K 31/4747, A61P 43/00

(54) **METHOD FOR SCREENING CYSTEINE PROTEASE INHIBITOR**

(30) Priority: 19.03.1999 JP 7572499
(71) Applicant: YAMANOUCHI PHARMACEUTICAL CO. LTD., Tokyo 103-8411 (JP)
(72) Inventor: OHMORI, Junya, Yamanouchi Pharmaceutical Co.,, Tsukuba-shi, Ibaraki 305-8585 (JP); OHTSUKA, Tomokazu, Yamanouchi Pharmaceutical Co.,, Tsukuba-shi, Ibaraki 305-8585 (JP); YATSUGI, Shin-ichi, Yamanouchi Pharmaceutical Co.,, Tsukuba-shi, Ibaraki 305-8585 (JP); OHNO, Kazushige, Yamanouchi Pharmaceutical Co.,Ltd, Tsukuba-shi, Ibaraki 305-8585 (JP)
(74) Representative: Geering, Keith Edwin
(86) International application number: JP0001662
(87) International publication number: WO0056918

(57) **Abstract**

A method for screening a cysteine protease inhibitor of which cysteine protease inhibitory activity is enhanced in the presence of endogenous polyvalent metal ion, which comprises treating a cysteine protease with a test compound in the presence of the polyvalent metal ion, and a pharmaceutical composition for treating apoptosis-associated diseases, which contains as the active ingredient a cysteine protease inhibitor of which cysteine protease inhibitory activity is enhanced in the presence of endogenous polyvalent metal ion.

SEQUENCE LISTING

<110> Yamanouchi Pharmceutical Co., Ltd.

<120> A screening method for a cysteine protease inhibitor

<130> Y9905

<150> JP P1999-075724

<151> 1999-03-19

<160> 3

<210> 1

<211> 4

<212> PRT

<213> Artificial Sequence

<220>

<223> Artificial ligand for cysteine protease

<400> 1

Tyr Val Ala Asp

1 5

<210> 2

<211> 4

<212> PRT

<213> Artificial Sequence

<220>

<223> Artificial ligand for cysteine protease

<400> 2

Asp Glu Val Asp

1 5

<210> 3
<211> 4
<212> PRT
<213> Artificial Sequence
<220>
<223> Artificial ligand for cysteine protease
<400> 3
Leu Glu His Asp
1               5

## Description

### Technical Field

The present invention relates to a screening method for a cysteine protease inhibitor of which cysteine protease inhibitory activity is enhanced in the presence of endogenous polyvalent metal ion, especially a cysteine protease inhibitor which substantially inhibits the cysteine protease in the presence of endogenous polyvalent metal ion, which comprises treating a cysteine protease (a thiol protease) with a test compound in the presence of the polyvalent metal ion.

The invention further relates to a pharmaceutical composition for inhibiting apoptosis, which contains as the active ingredient a cysteine protease inhibitor of which cysteine protease inhibitory activity is enhanced in the presence of endogenous polyvalent metal ion, especially a cysteine protease inhibitor which substantially inhibits a cysteine protease in the presence of endogenous polyvalent metal ion.

### Background of the Invention

Cathepsin, calpain, caspase, rhinovirus 3C protease, and the like is known as the proteases classified as cysteine proteases, and it has been reported that they regulate various physiological functions such as bone metabolism, immunity, inflammation, and/or programmed cell death.

For example, with regard to caspase, 13 or more kinds of isozymes are known in mammals until now (Thornberry, et al., Science 281, pp1321, 1998), and these are usually present in an inactive state. Among them, caspase 1 (interleukin-1β converting enzyme, ICE) is essential for the production of inflammatory cytokines and is considered to play an important role in various inflammatory responses. On the other hand, the roles as apoptosis- executioner molecules or apoptosis signal-transducing molecules are clearly shown for caspase 3 (CPP32, Yama, apopain), caspase 8 (MACH, FLICE, Mch5), caspase 9 (ICE-LAP6, Mch6), caspase 2, caspase 6, caspase 7, caspase 10, and caspase 12 (Thornberry, et al., Science 281, pp1321, 1998; Nakagawa et al., Nature 403, pp98, 2000). It is considered that these caspases are activated by an apoptotic signal (Kerr et al., Br. J. Cancer 26, pp239, 1972; Wyllie eds., "Apoptosis" Br. Med. Bull. vol. 53, 1997; Al-Rubeai eds., "Apoptosis" Adv. Biochem. Eng. Biotech. vol. 62, 1998), and they are involved in a process of apoptosis through the processing of proenzymes of other caspase family and other various substrate proteins.

Apoptosis is generally regarded as a programmed cell death, and is associated with, for example, degenerative diseases such as Alzheimer's disease, Parkinson's disease or Huntington's chorea; ischemic diseases such as cerebral infarction or myocardial infarction; osteomyelodysplasia such as aplastic anemia; or osteoarthritis. As its distinct feature, the cell death is considered to occur along with the cleavage of DNA in nucleosome units, and the cleavage of DNA is assumed to be an indicator of apoptosis (Wyllie, A. H., Nature, 284, pp555, 1980).

Moreover, it is also known that an increased activity of a cysteine protease is associated with various diseases, and a possibility that the inhibition of the activated cysteine protease in these diseases is effective for the treatment and prevention of these diseases is shown (a special issue of Gendai Kagaku, "Puroteaze no Seitaikinou (Vital Functions of Protease)", Tokyo Kagaku Dojinn, 1993; "Puroteaze to Sono Inhibita (Protease and its inhibitor)", Medical View, 1993; "Noukyoketsu to Puroteaze (Cerebral Ischemia and Protease)", Jikken Igaku 15(17), pp2063, 1997; "Kasupaze to Apotosisu (Caspase and Apoptosis)", Jikken Igaku 15(17), pp2071, 1997; MaKerrow et al., eds., "Cysteine proteases" Perspect. Drug Discov. Des. Vol 6, 1996; Leung et al., J. Med. Chem., 43, pp305, 2000, and so forth).

For example, the activation of caspase has been observed in various pathologic models of cerebral ischemia (Binhui et al., J. Cereb. Blood Flow Metab. 18, pp248, 1998; Asahi et al., J. Cereb. Blood Flow Metab. 17, pp11, 1997; Namura et al., J. Neurosci. 15, pp3659, 1998; Barinaga, Science 281, pp302, 1998), and a peptidyl caspase inhibitor exhibits a reduction of infarction (Hara et al., Proc. Natl. Acad. Sci. U.S.A. 94, pp2007, 1997; Hadingham et al., Br. J. Pharmacol. 122, suppl. 428P, 1997). Moreover, in a similar pathologic model using a caspase 1-knockout mouse, reduction of cerebral infarction and the improvement of neurological deficits have been reported (Schielke et al., J. Cereb. Blood Flow Metab. 18, pp180, 1998). Furthermore, it is proved that caspase acts an important role in amyotrophic lateral sclerosis, Alzheimer's disease, and Huntington's chorea (Hartmann et al., Proc. Natl. Acad. Sci. U.S.A. 97, pp2875, 2000; Gervais et al., Cell 97, pp395, 1999; Ona et al., Nature 399, pp263, 1999; Friedlander et al., Nature 388, pp31, 1997; Kim et al., Science 277, pp 373, 1997). In a myocardial infarction model and a certain hepatopathy model, it is also shown that a peptidyl caspase inhibitor reduces infarction and improves heart function (Yaoita et al., Circulation 97, pp276, 1998), and it also prevents acute hepatic failure (Kubo et al., Proc. Natl. Acad. Sci. U.S.A. 95, pp9552, 1998). In addition, it is also shown that the activation of caspase is associated with some types of renal diseases and autoimmune diseases (Ali et al., Am. J. Physiol. Regul. Integr. Comp. Physiol., 278(3), R763-R769, 2000; Ingelsson et al., Immunology Lett., 63, pp125, 1998; Craighead et al., J. Neurosci. Res. 57, pp236, 1999; Zheng et al., Nature, 400, pp410, 1999; Mitra et al., Am. J. Hematology, 59, pp279, 1998). Accordingly, when a caspase inhibitor having suitable physical properties for clinical use as a medical agent can be developed, it would be useful as a therapeutic agent for these degenerative diseases, autoimmune diseases or apoptosis-associated diseases (Talanian et al., Annu. Rep. Med. Chem. 33, pp273, 1998; Chinnaiyan et al., Curr. Biol. 6, pp555, 1996; Cohen et al., Biochem. J. 326, pp1, 1997; Garcia-Calvo et al., J. Biol. Chem. 273, pp32608, 1998).

When such an inhibitor, having appropriate properties for use as a medical agent, against a suitable cysteine protease associated with the diseases can be developed, it would be useful as a therapeutic agent for the diseases. Accordingly, experimental methods for finding out inhibitors of various cysteine proteases including caspase has been currently reported.

For example, a spectroscopic detection method using a synthetic peptide having a sequence equal or similar to the cleavage site of a substrate and labeled with a fluorescent or coloring compound, such as amino-4-methylcoumalin (AMC), p-nitroaniline (pNA) or the like as the substrate, is frequently used (Knight, Methods in Enzymology 248, pp18, Academic Press, 1995). In addition, a method of detecting the proteolysis of a substrate protein using a radioactive isotope, a detection method using an antibody, and the like are also known (Thornberry, Methods in Enzymology 244, pp615, Academic Press, 1994). For example, in the case of measuring the activity of caspase 1, a spectroscopic measurement method is frequently used wherein peptidyl-MCA or peptidyl-pNA obtainable by introducing a leaving group such as AMC or pNA to P1' site (immediately next to the amino acid of a cleavage site), i.e., Ac-Tyr-Val-Ala-Asp-MCA, Ac-Tyr-Val-Ala-Asp-pNA, or the like is used as a substrate. Other than the method, a method of detecting the cleavage reaction with labeling an interleukin 1β precursor (pro-IL-1β) with [³⁵S]methionine, or a detection method using an antibody of pro-IL-1β is applied (Thornberry, Nature 356, pp768, 1992; Thornberry, Methods in Enzymology 244, pp615, Academic Press, 1994). Moreover, in the case of caspase 3, the activity is frequently measured in a similar manner to the case of caspase 1 using Ac-Asp-Glu-Val-Asp-MCA, Ac-Asp-Glu-Val-Asp-pNA, or poly(ADP-ribose)polymerase (PARP) as a substrate (Nicholson et al., Nature 376, pp37, 1995). Furthermore, in the case of caspase 9, the activity is measured using Ac-Leu-Glu-His-Asp-MCA (Thornbery et al., J. Biol. Chem. 272, pp17907, 1997) or a precursor of caspase 3, 6, or 7 (Srinivasula et al., Mol. Cell 1, pp949, 1998) as a substrate.

Synthetic cysteine protease inhibitors commonly known until now include peptides and peptide analogues obtained from combinations of amino acids through try and error or by procedures of combinatorial chemistry, or derivatives obtained by chemical modification of partial structures of amino acid sequences of proteins which are recognized as substrates. These synthetic peptides and synthetic peptide analogues have any or plurality of problems of solubility or absorption, excretion, metabolism, or distribution or migrating ability to a target organ, so that it is entirely impossible to use them as therapeutic agents for clinical application.

In addition, there are protease inhibitors that exhibit the action through a covalently bonding reaction with a protease. These covalently bonding inhibitors have a characteristic of having a highly reactive functional group which becomes a point of action with a substrate protein, for example, an aldehyde group or an equivalent thereof or a precursor thereof (e.g., Ac-DEVD.CHO, Nature 376, pp37, 1995; MDL28170, Tred. in Bio. Sci. 16, pp150, 1991 and so forth), a leaving group such as a halogen atom, diazo group or an acyloxy group, or an epoxy group (e.g., z-VAD.FMK, Hara et al., Proc. Natl. Sci. U.S.A. 94, pp2007, 1997; E-64, Tred. in Bio. Sci. 16, pp150, 1991, and so forth), or a structure which becomes a Michael acceptor or a point of reaction of redox-type reaction (e.g., 6-hydroxytetranguol, J. Antibiot. 51, pp79, 1998 and so forth). Such inhibitors mostly have problems of specificity, stability, antigenicity, toxicity, and the like.

As a method for solving the problems, it is required to find out an inhibitor having an entirely different skeletal structure. Heretofore, non-peptidyl inhibitors obtained by modifying synthetic peptide and synthetic peptide analogues have been known but there is only a few wherein the structure can be modified so as to solve the above problems substantially. Especially, with regard to cysteine proteases, there is present no inhibitor which solves these problems to a large extent (J. Med. Chem. 43, pp305, 2000).

The development of a cysteine protease inhibitor which solves the above problems is an important issue in this field, and a novel screening method capable of finding out a cysteine protease inhibitor of which inhibitory activity cannot be found out or is evaluated to be only weak by a conventional screening method, although it do work under physiological conditions, is a useful technology which has a potential for solving the present problems.

On the other hand, it is known that several polyvalent metal ions are exist endogenously. Zinc ion is an ion which distributes widely and in a relatively large amount in a living body among the polyvalent metal ions. The ion is contained in an amount of about 1.4 to 2.3 g in a human body of 70 kg, the value corresponding to a half of that of iron, 10 to 15 times of that of copper, and about 100 times of that of manganese. It is contained in 300 or more types of proteins and also stored in endoplasmic reticulum in a cell, as well as it is retained loosely in a metallothionein and a cell skeleton. Further, the average zinc concentration in a brain is 10 µg/g-wet weight. It is also shown that an ion channel for the passage of zinc ion is present on a cell membrane and zinc ion is released from a nerve terminal by neuronal excitation (Baiometaru (Biometal), Kurobune Syuppan, 1998; Cuajungco et al., Neurobiology of Disease 4, pp137, 1997; Lee et al., Nature 399, suppl. A7, 1999).

Although a serine protease inhibitor is known as a protease inhibitor through the intervention of such zinc ion distributed in a living body (Kats, B. A. et al., Nature 391, pp608, 1998), a cysteine protease inhibitor through the intervention of a endogenous polyvalent metal ion and a screening method thereof are not known.

### Disclosure of the Invention

As a result of the extensive studies under such a technical level, the present inventors have found that the screening of a cysteine protease inhibitor in the presence of a endogenous polyvalent metal ion unexpectedly affords a screening method capable of finding out a cysteine protease inhibitor whose inhibitory activity is evaluated to be weak or cannot be found out by a conventional screening method.

Furthermore, we have found that a cysteine protease inhibitor of which cysteine protease inhibitory activity is enhanced in the presence of endogenous polyvalent metal ion has an apoptosis inhibitory activity.

Namely, an object of the invention is to provide a screening method for a cysteine protease inhibitor of which cysteine protease inhibitory activity is enhanced in the presence of endogenous polyvalent metal ion, especially a cysteine protease inhibitor which substantially inhibits the cysteine protease in the presence of endogenous polyvalent metal ion.

Moreover, an object of the invention is to provide a pharmaceutical composition which contains a cysteine protease inhibitor found out by the screening method.

Further, an object of the invention is to provide a cysteine protease inhibitor of which cysteine protease inhibitory activity is enhanced in the presence of endogenous polyvalent metal ion, especially a cysteine protease inhibitor which substantially inhibits the cysteine protease in the presence of endogenous polyvalent metal ion.

Furthermore, an object of the invention is to provide a pharmaceutical composition for inhibiting apoptosis, which contains the cysteine protease inhibitor as the active ingredient.

In the invention, "a cysteine protease inhibitor which substantially inhibits the cysteine protease" means a cysteine protease inhibitor which has no or weak inhibitory activity against a cysteine protease in the absence of a polyvalent metal ion but has a sufficient inhibitory activity in the presence of the polyvalent metal ion.

By using the present screening method, it is possible to find out a cysteine protease inhibitor whose inhibitory activity is evaluated to be weak or cannot be found out by a conventional screening method, especially a cysteine protease inhibitor having a skeletal structure different from that of a conventional cysteine protease inhibitor. The inhibitor thus found out has little problem in clinical use, possesses no highly reactive functional group, and also has non-peptide structure.

The screening method of the invention is a method for finding out a compound of which cysteine protease inhibitory activity is enhanced in the presence of endogenous polyvalent metal ion by promptly screening a number of test compounds, and can be carried out by adding a endogenous polyvalent metal ion to the system of a screening method hitherto known.

For example, it is possible to determine whether a test compound is a protease inhibitor or not by mixing the test compound with a cysteine protease and a labeled substrate of the cysteine protease and measuring the processing amount or processing rate of the substrate by the action of the cysteine protease. Also, a compound of which inhibitory activity is enhanced in the presence of the polyvalent metal ion can be determined by comparing the decomposed amounts or decomposing rates of the substrate in the presence and absence of the polyvalent metal ion.

Furthermore, in the screening method of the invention, the mode of inhibition of the cysteine protease inhibitor can be determined by measuring the cysteine protease inhibitory activities of the test compound at various concentrations of the test compound and/or the substrate.

As the cysteine protease usable in the screening method of the invention, for example, caspase and the like may be mentioned. Preferred is caspase 1, 3, 6, 7, 8, 9 or 12, and particularly preferred is caspase 3. These cysteine proteases are available by preparation according to the method described in the above literatures or by purchase. Moreover, the cysteine protease can be used at various concentrations, and for example, preparations having adjusted concentrations of 1 ng/mL to 100 µg/mL, 10 pM to 100 nM, or 0.1 unit to 10,000 units can be used in the present screening method.

In the invention, " endogenous polyvalent metal ion " means a metal ion having two or more valency and endogenously existing, and preferred is a divalent or trivalent metal ion.

The endogenous polyvalent metal ion usable in the screening method of the invention includes zinc ion, iron ion, copper ion, selenium ion, chromium ion, cobalt ion, manganese ion, or the like. Preferred is zinc ion and particularly preferred is divalent zinc ion. Moreover, the polyvalent metal ion can be used at various concentrations, and for example, preparations having adjusted concentrations of 1 nM to 50 mM can be used in the present screening method.

As the substrate of a cysteine protease usable in the screening method of the invention, hitherto known substrates may be mentioned, which include Ac-Tyr-Val-Ala-Asp-MCA, Ac-Tyr-Val-Ala-Asp-pNA, pro-IL-1β, Ac-Asp-Glu-Val-Asp-MCA, Ac-Asp-Glu-Val-Asp-pNA, PARP, Ac-Leu-Glu-His-Asp-MCA, a precursor of caspase 3, 6 or 7, or the like, for example. Moreover, these substrates can be used at various concentrations, and for example, preparations having adjusted concentrations of 1 µM to 100 µM in the case of synthetic substrates, and of 1 fM to 10 mM in the case of proteins can be used in the present screening method.

As the labeling for use in the screening method of the invention, hitherto known labeling may be mentioned, which include fluorescence, coloring, irradiation, antigen-antibody reaction, or the like.

As the enzymatic reaction liquid usable in the screening method of the invention, a known liquid may be utilized. For example, an enzymatic reaction liquid containing a metal chelator such as EDTA; a surfactant such as saccharose, 3-[(3-cholamidopropyl)dimethylammonio]-1-propane sulfonate, bovine serum albumin, or glycerol; a reducing agent such as dithiothreitol or 2-mercaptoethanol; HEPES, and the like is adjusted to pH 6 to 8.5 and can be used in the screening method of the invention under a condition of 0 to 45°C.

As the test compound usable in the screening method of the invention, a newly synthesized compound, a commercial compound or a known compound which is registered in a chemical file but the various activities are unknown, a series of compounds obtained by the technology of combinatorial chemistry can be used. Also, a supernatant of culture of a microorganism, a natural component derived from a plant or a marine organism, an animal tissue extract, and the like can be used. In addition, a compound obtainable by chemically modifying a compound found out in the screening method of the invention can be also used. These test compounds can be used at various concentrations in the screening method of the invention. For example, preparations having adjusted concentrations of 1 nM to 200 µM can be used in the present screening method.

A cysteine protease inhibitor inhibiting 50% or more of a cysteine protease activity at a concentration of 100 µM or less in the presence of a endogenous polyvalent metal ion can be find out by the use of the screening method of the invention.

A cysteine protease inhibitor inhibiting 50% or more of apoptosis at a concentration of 100 µM or less can be find out by the use of the screening method of the invention.

As the cysteine protease inhibitor of the invention, for example, a caspase inhibitor and the like may be mentioned. Preferred is an inhibitor of caspase 1, 3, 6, 7, 8, 9 or 12, and particularly preferred is a caspase 3 inhibitor.

The inhibitory mode of the cysteine protease inhibitor of the invention is a competitive or non-competitive inhibition, and preferred is a competitive inhibition.

The cysteine protease inhibitor of the invention means a compound found out by the screening method of the invention, which inhibits the activity of a cysteine protease in vivo or in vitro. Unlike conventional inhibitors, cysteine protease inhibitors through the intervention of zinc ion also include non-peptidyl inhibitors having no functional group serving as a point of covalently bonding reaction, owing to the characteristic mode of inhibition. These inhibitors are expected to overcome the problems derived from the structure of peptides, peptide analogues or peptide mimetics or the structure having a reactive functional group, especially the problems of physical properties, the problems being limitations for the use of conventional cysteine protease inhibitors as therapeutic agents.

Examples of the cysteine protease inhibitor of the invention include bis (1H-benzimidazol-2-yl)methane, bis (1H-benzimidazol-2-yl)ketone oxime, 1,5'-dipropylspiro [1,8-naphthyridine-3(2H),2'-[1,3]oxathiolo[4,5-c][1,8]naphthyridine]-2,4,4'(1H,5'H)-trione, 1,5'-dipropylspiro[1,3]oxathiolo[4,5-c]quinoline-2,3'(2H)-quinoline]-2,4,4'(1H,5'H)-trione, or the like, and a similar activity can be shown in the compounds having different structures. Preferred cysteine protease inhibitor is 1,5'-dipropylspiro[1,8-naphthyridine-3(2H),2'-[1,3]oxathiolo[4,5-c][1,8]naphthyridine]-2,4,4'(1H,5'H)-trione or 1,5'-dipropylspiro[1,3]oxathiolo[4,5-c]quinoline-2,3'(2H)-quinoline]-2,4,4'(1H,5'H)-trione, and particularly preferred is 1,5'-dipropylspiro[1,8-naphthyridine-3(2H),2'-[1,3]oxathiolo[4,5-c][1,8]naphthyridine]-2,4,4'(1H,5'H)-trione.

A cysteine protease inhibitor of the invention, 1,5'-dipropylspiro[1,8-naphthyridine-3(2H),2'-[1,3]oxathiolo[4,5-c][1,8]naphthyridine]-2,4,4'(1H,5'H)-trione can be produced starting with methyl 2-(propylamino)nicotinate according to the method described in Example 1, and 1,5'-dipropylspiro[1,3]oxathiolo[4,5-c]quinoline-2,3'(2H)-quinoline]-2,4,4'(1H,5'H)-trione can be produced starting with N-propylaniline according to the method described in Example 2.

There are cases that the cysteine protease inhibitors of the invention can form acid addition salts or salts with bases. The compounds of the invention also include these salts. Concrete examples of such salts include acid addition salts formed with inorganic acids such as hydrochloric acid, hydrobromic acid, hydroiodic acid, sulfuric acid, nitric acid, phosphoric acid, etc.; organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, etc.; and acidic amino acids such as aspartic acid, glutamic acid, etc.; and salts with inorganic bases such as sodium, potassium, magnesium, calcium, aluminum, etc.; and organic bases such as methylamine, ethylamine, monoethanolamine, diethanolamine, triethanolamine, cyclohexylamine, lysine, ornithine, etc.

Furthermore, the cysteine protease inhibitors or pharmaceutically acceptable salts thereof may be isolated as various types of solvated products such as hydrates or adducts with ethanol or as substances of crystalline polymorph, and the compounds of the invention also include such various hydrates, solvates, or substances of crystalline polymorph.

The apoptosis inhibitory action of the cysteine protease inhibitor of the invention is effective in various cells. Preferred is neuronal cells, and particularly preferred is a hippocampal neuron.

The pharmaceutical composition containing the cysteine protease inhibitor of the invention as the active ingredient is useful for the various diseases caused by increased activity of a cysteine protease such as caspase 3. For example, the pharmaceutical composition of the invention is useful for apoptosis-associated diseases, preferably neuronal apoptosis- associated diseases, neurodegenerative diseases such as Alzheimer's disease or Parkinson's disease, or cerebral ischemic diseases such as cerebral infarction, particularly preferably neurodegenerative diseases such as Alzheimer's disease which are associated with apoptosis of a hippocampal neuron.

### Industrial Applicability

According to the screening method of the invention, it is possible to find out a cysteine protease inhibitor which cannot be found out by a conventional screening method. For example, unlike conventional inhibitors, cysteine protease inhibitors through the intervention of zinc ion also include non-peptidyl inhibitors having no functional group serving as a point of covalently bonding reaction, owing to the characteristic mode of inhibition thereof. These inhibitors are expected to overcome the problems derived from the structure of peptides, peptide analogues or peptide mimetics or the structure having a reactive functional group, especially the problems of physical properties, the problems being limitations for the use of conventional cysteine protease inhibitors as therapeutic agents.

For example, the inhibitory activity of bis(1H-benzimidazol-2-yl) methane, bis (1H-benzimidazol-2-yl) ketone oxime, 1,5'-dipropylspiro[1,8-naphthyridine-3(2H),2'-[1,3]oxathiolo[4,5-c][1,8]naphthyridine]-2,4,4'(1H,5'H)-trione, and 1,5'-dipropylspiro[1,3]oxathiolo[4,5-c]quinoline-2,3'(2H)-quinoline]-2,4,4'(1H,5'H)-trione obtained by the screening method of the invention is remarkably enhanced by the presence of zinc. Thus, a similar activity can be shown in compounds having different structures. These findings are obtained for the first time with regard to cysteine protease inhibitors. Furthermore, unlike known inhibitors such as z-VAD.FMK which exhibit no enhanced inhibitory action by the presence of zinc, the compounds obtained by the screening method of the invention have no reactive functional group and are low molecular weight non-peptidyl compounds, and their skeletal structures are much different from those of conventional cysteine protease inhibitors. It is highly likely that such low molecular weight non-peptidyl compounds are more useful for clinical use than the peptide analogues, the peptide mimetics or the conventional cysteine protease inhibitors having a reactive functional group.

In particular, 1,5'-dipropylspiro[1,8-naphthyridine-3(2H),2'-[1,3]oxathiolo[4,5-c][1,8]naphthyridine]-2,4,4'(1H,5'H)-trione and 1,5'-dipropylspiro[1,3]oxathiolo[4,5-c]quinoline-2,3'(2H)-quinoline]-2,4,4'(1H,5'H)-trione, which show substantially no caspase 3 inhibitory activity in the absence of zinc but show the inhibitory activity in the presence of zinc, exhibit a suppressing action against DNA fragmentation induced by apoptosis in an apoptosis-inducing experiment using rat hippocampal primary culture cell. Thus, an apoptosis-suppressing action was observed in compounds having different skeletal structures. Accordingly, 1,5'-dipropylspiro[1,8-naphthyridine-3(2H),2'-[1,3]oxathiolo[4,5-c][1,8]naphthyridine,]-2,4,4'(1H,5'H)-trione and 1,5'-dipropylspiro[1,3]oxathiolo[4,5-c]quinoline-2,3'(2H)-quinoline]-2,4,4'(1H,5'H)-trione are considered to express their enzyme-inhibitory activity through the intervention of zinc ion in a cell. Therefore, they are expected to be useful for treating and preventing various diseases caused by an increased activity of a cysteine protease such as caspase 3.

As described above, the cysteine protease inhibitor of the invention of which cysteine protease inhibitory activity is enhanced in the presence of endogenous polyvalent metal ion and the pharmaceutical composition containing the cysteine protease inhibitor and a pharmaceutically acceptable carrier are useful for the various diseases caused by an increased activity of a cysteine protease such as caspase 3, for example, apoptosis-associated diseases. Furthermore, since the pharmaceutical composition of the invention is effective against apoptosis of rat hippocampal cells, it is useful for neuronal apoptosis-associated diseases, for example, neurodegenerative diseases such as Alzheimer's disease or Parkinson's disease, or cerebral ischemic diseases such as cerebral infarction, particularly neurodegenerative diseases such as Alzheimer's disease which are associated with apoptosis of a hippocampal neuron.

The pharmaceutical composition containing the cysteine protease inhibitor of the invention as the active ingredient can contain pharmaceutically acceptable carrier(s), and is formulated to tablets, powders, fine granules, granules, capsules, pills, liquids, injections, suppositories, ointments, cataplasms, and the like, and the composition is administered orally (including sublingual administration) or parenterally.

The clinical dose of the cysteine protease inhibitor of the invention toward human is optionally determined individually in consideration of the symptoms, body weight, age, and sexuality of the patient to be applied, application route, and the like, but in general, it is administered, per one adult, orally in an amount of 0.1 to 5000 mg/day, preferably 1 to 500 mg/day once to several times a day, dividedly, or intravenously in an amount of 0.1 to 5000 mg/day, preferably 1 to 500 mg/day once to several times a day, dividedly, or intravenously for 1 to 24 hours per day continuously. Of course, as described above, since the dose varies depending on various conditions, a lesser amount than the above dose is sufficient in some cases.

As solid compositions for oral administration according to the invention, tablets, powders, granules, and the like are used. In such solid compositions, one or more active substances are mixed with at least one inactive diluent, for example, lactose, mannitol, glucose, hydroxypropyl cellulose, microcrystalline cellulose, starch, polyvinyl pyrrolidine, or magnesium metasilicic aluminate. The compositions may contain additives other than the inert diluent, for example, lubricants such as magnesium stearate, disintegrators such as cellulose calcium glycolate, stabilizers such as lactose, solubilizers such as glutamic acid or aspartic acid according to a usual method. The tablets or pills my be coated with sugar or a film soluble in stomach or intestines, such as sucrose, gelatin, hydroxypropyl cellulose, hydroxypropylmethyl cellulose phthalate, and the like, if necessary.

Liquid compositions for oral administration contain pharmaceutically acceptable emulsifiers, solvents, suspending agents, syrup agents, elixir agents, or the like, and inert diluents, for example, purified water or ethanol. The compositions may contain auxiliaries such as solubilizers or dissolution aids, wetting agents, or suspending agents, sweeteners, flavors, fragrances, and antiseptic agents.

The injections for parenteral administration contain sterile aqueous or non-aqueous solvents, suspending agents, and emulsifiers. Examples of the aqueous solvents and suspending agents include distilled water for injections and physiological saline. Examples of the non-aqueous solvents and suspending agents include propylene glycol, polyethylene glycol, vegetable oils such as olive oil, alcohols such as ethanol, polysorbate 80 (a trade name), and the like. Such compositions may further contain additives such as isotonicities, antiseptic agents, wetting agents, emulsifiers, dispersants, stabilizers (e.g., lactose), and solubilizers or dissolution aids. These are sterilized by filtration through a bacteria-retaining filter, mixing with a bactericide, or irradiation. These may be used by preparing sterile solid compositions and dissolving them into sterile water or sterile solvents for injections before use.

### Best Mode for Carrying Out the Invention

The following will explain the present invention in detail with reference to Examples. However, the invention may be any of the screening methods for a cysteine protease wherein a polyvalent metal ion in a living body is added and the cysteine protease inhibitors found out by the method, and is not limited to these Examples.

### Reference Example

### Preparation of protease: Preparation of recombinant human caspase 3 protein

From human brain cDNA, the N-terminal region encoding a protein with a molecular weight of about 17 kDa (p17) and C-terminal region encoding a protein with a molecular weight of about 12 kDa (p12) were amplified by PCR, and then subcloned into an Escherichia coli expression plasmid (pET3a). Using these plasmids, a host bacterium for expressing proteins (BL21 (DE3) pLysS) was transformed. Then, the transformed bacterium was cultured in M9 medium and an active caspase 3 was prepared. Firstly, fungus body recovered from M9 medium was broken by sonication and an inclusion body was recovered by centrifugation. The inclusion body was solubilized under denatured conditions using 3M guanidine solution, and after mixing with p17 polypeptide and p12 polypeptide, diluted with a dialysis solution. The whole was left at room temperature for 1 hour at this situation, and after regeneration, concentrated. After the sample was dialyzed to exchange the buffer solution, insoluble proteins were removed by ultracentrifugation (100,000 X G, 30 minutes). Upon quantitative protein determination, about 30 mg of a protein was recovered.

### Example 1

### Synthesis of 1,5'-dipropylspiro[1,8-naphthyridine-3(2H),2'-[1,3]oxathiolo[4,5-c][1,8]naphthyridine]-2,4,4'(1H,5'H)-trione

1) Into a mixture of acetic anhydride (20 mL) and acetic acid (10 mL) was added 1.46 g of methyl 2-(N-propylamino)nicotinate (Martin Winn et al., J. Med. Chem., 36, 2676-2688 (1993)), followed by heating under reflux for overnight. The reaction solution was concentrated under reduced pressure and the resulting residue was purified by silica gel column chromatography (chloroform-methanol) to obtain methyl 2- (N-acetyl-N-propylamino)nicotinate (1.71 g).
   ¹H-NMR (CDCl₃: δ from TMS): 8.71 (1H, dd), 8.32 (1H, dd), 7.42 (1H, ddd), 3.92 (3H, s), 3.87 (2H, s), 1.80 (3H, s), 1.55 (2H, se), 0.87 (3H, t)
2) Methyl 2-(N-acetyl-N-propylamino)nicotinate (1.32 g) was dissolved in tetrahydrofuran (50 mL), and potassium t-butoxide (0.69 g) was added thereto. The whole was stirred at room temperature for 30 minutes and heated under reflux for further 1 hour. The reaction liquid was cooled on standing to room temperature and 1N hydrochloric acid (7 mL) was added under stirring, followed by further addition of water (100 mL). The resulting precipitate was collected by filtration and, after washing with water, dried under reduced pressure to obtain 4-hydroxy-1-propyl-1,8-naphthyridin-2(1H)-one (0.70 g) as a slightly orange solid. ¹H-NMR (DMSO-d₆; δ from TMS): 11.61 (1H, d), 8.64 (1H, dt), 8.23 (1H, dt), 7.27 (1H, ddd), 5.89 (1H, d), 4.26 (2H, td), 1.61 (2H, se), 0.89 (3H, td)
3) In accordance with the reaction conditions of Ziegler, E. et al. (Monatsh Chem., 96, 1030-1035 (1965)), 4-hydroxy-1-propyl-1,8-naphthyridin-2(1H)-one (0.66 g) was suspended in a mixture of 1,4-dioxane (2 mL) and thionyl chloride (10 mL), followed by heating under reflux for 1 hour. The reaction liquid was cooled on standing to room temperature and concentrated under reduced pressure. To a yellow solid obtained as the residue was added chloroform, and insoluble matter was filtered off. The filtrate was concentrated under reduced pressure, and chloroform and methanol were added to the resulting yellow solid, followed by further concentration under reduced pressure. After the addition of chloroform, suspending matter was collected by filtration and, after washing with chloroform and methanol, dried under reduced pressure to obtain bis(4-hydroxy-2-oxo-1-propyl-1,2-dihydro-1,8-naphthyridin-3-yl) sulfide (0.20 g).
   mp: 250-251°C
4) Bis(4-hydroxy-2-oxo-1-propyl-1,2-dihydro-1,8-naphthyridin-3-yl) sulfide (0.19 g) and manganese dioxide (0.38 g) were suspended in N,N-dimethylformamide (5 mL), followed by stirring under heating for overnight. The reaction liquid was filtered through celite and the filtrate was concentrated under reduced pressure. The resulting residue was purified by silica gel column chromatography (chloroform-methanol) and, after washing with a small amount of methanol, dried under reduced pressure to obtain 1,5'-dipropylspiro[1,8-naphthyridine-3(2H),2'-[1,3]oxathiolo[4,5-c][1,8]naphthyridine]-2,4,4'(1H,5'H)-trione (85 mg) as a yellow solid.
   mp: 248-249°C
   ¹H-NMR (CDCl₃; δ from TMS) : 8.66 (2H, td), 8.30 (1H, dd), 8.19 (1H, dd), 7.25 (1H, dd), 7.22 (1H, dd), 4.45 (2H, m), 4.32 (1H, ddd), 4.15 (1H, ddd), 1.70-1.80 (4H, m), 1.00 (6H, td).

### Example 2

### Synthesis of 1,5'-dipropylspiro[1,3]oxathiolo[4,5-c]quinoline-2,3'(2H)-quinoline]-2,4,4'(1H,5'H)-trione

1) To polyphosphoric acid prepared by a usual method starting with phosphoric acid (4.3 g) and diphosphorus pentoxide (7.7 g) were added N-propylaniline (2.7 g) and diethyl malonate (4 ml), followed by stirring under heating at 170°C for 30 minutes and further at 200°C for 30 minutes. The reaction liquid was cooled and, after the addition of ice-water and diethyl ether, the whole was stirred. The precipitate was collected by filtration and, after washing with water and ethyl acetate, was recrystallized from methanol to obtain 4-hydroxy-1-propylquinolin-2(1H)-one (460 mg).
   ¹H-NMR (DMSO-d₆; δ from TMS): 11.32 (1H, brs), 7.90 (1H, d), 7.61 (1H, t), 7.50 (1H, d), 7.22 (1H, t), 5.86 (1H, s), 4.12 (2H, t), 1.52-1.65 (2H, m), 0.93 (3H, t).
2) 1,5'-dipropylspiro[1,3]oxathiolo[4,5-c]quinoline-2,3'(2H)-quinoline]-2,4,4'(1H,5'H)-trione was obtained in a similar manner to articles 3 and 4 of Example 1 starting with 4-hydroxy-1-propylquinolin-2(1H)-one.
   MS (FAB, M+1): 435
   ¹H-NMR (CDCl₃; δ from TMS) : 8.03 (1H, dd), 7.96 (1H, dd), 7.69 (1H, td), 7.59 (1H, td), 7.38 (1H, d), 7.30 (1H, t), 7.25 (1H, t), 7.18 (1H, d), 4.18-4.27 (2H, m), 4.07-4.18 (1H, m), 3.86-3.97 (1H, m), 1.70-1.84 (4H, m), 1.04 (6H, t).

### Example 3

### Screening method of caspase 3 inhibitor

A number of compounds were used as test compounds for the present screening method. Among them, commercial products (Salor Co.) were used in the cases of bis(1H-benzimidazol-2-yl)methane (Compound A) and bis(1H-benzimidazol-2-yl)ketone oxime (Compound B). In addition, 1,5'-dipropylspiro[1,8-naphthyridine-3(2H),2'-[1,3]oxathiolo[4,5-c][1,8]naphthyridine]-2,4,4' (1H,5'H)-trione (Compound C) synthesized in Example 1 and 1,5'-dipropylspiro[1,3]oxathiolo[4,5-c]quinoline-2,3'(2H)-quinoline]-2,4,4'(1H,5'H)-trione (Compound D) synthesized in Example 2 were used as test compounds. 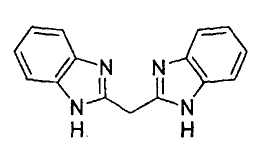 bis (1H-benzimidazol-2-yl)methane: Compound A 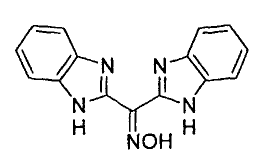 bis(1H-benzimidazol-2-yl)ketone oxime: Compound B

Furthermore, as a control compound, a known caspase 3 inhibitor (Proc. Natl. Acad. Sci. U.S.A. 94, pp2007, 1997, etc.), z-VAD.FMK (z-Val-Ala-Asp(OMe)-CH₂F) was used (a commercial product, Takara Shuzo Co., Ltd., SP020).

### 1) In the absence of zinc ion

Solution A comprising composition of 50 mM HEPES, pH 7.5, 10% (w/v) saccharose, and 0.1% (w/v) 3-[(3-cholamidopropyl)dimethylammonio]-1-propane sulfonate (CHAPS) was used as the base of a liquid for enzymatic reaction with adding a test compound, if necessary. A 10 ng/100 µL human caspase 3 solution (Solution B: Solution A + 10 mM dithiothreitol (DTT)) and a test compound at different concentrations were mixed and the mixture was left at room temperature for 30 minutes. In a control experiment, caspase 3 mixed with DMSO instead of the test compound was used. Then, 100 µL of 50 µM Ac-Asp-Glu-Val-Asp-MCA (Solution A, Peptide Kenkyusyo, 3171-v) as a substrate solution were added thereto to start reaction. Time course of the decomposition of the substrate were measured on a fluorescent photometer (Corona Denki, MTP-32, excitation wavelength/360 nm, emission waverangeth/450 nm or SLT, fluostar, excitation wavelength/380 nm, emission wavelength/460 nm) and an enzyme activity of the test compound-added group relative to the control was calculated.

### 2) In the presence of zinc ion

The effects of the test compound at various concentrations on the enzyme activity were measured in a similar manner to the case in the absence of zinc ion as described in the section 1). However, a substrate solution containing 3 mM ZnSO₄ was used.

### 3) Detection of increase of the caspase 3 inhibitory activity of a test compound when zinc ion was added

The enzyme activities calculated in the absence of zinc ion (-Zn) and in the presence of zinc ion (+Zn) were compared (enzyme activity (+Zn)/enzyme activity (-Zn)).

### 4) Screening results of the test compounds

As a result of screening a number of compounds according to the screening method of the above 1), 2) and 3), it was confirmed that the caspase 3 inhibitory activities of four compounds of Compounds A to D were enhanced in the presence of zinc ion. In the cases of Compounds A and B, the experiments were carried out under conditions that the enzyme solution contained 25 ng/100 µL human caspase 3, the concentration of a test compound was 100 µM, and the reaction time was 15 minutes. At that time, the ratios (enzyme activity (+Zn)/enzyme activity (-Zn)) for Compounds A and B were 0.37 and 0.44, respectively. Moreover, in the case of Compound C, the enzyme activity was inhibited by only 17% at 100 µM in the absence of zinc ion, but IC₅₀ was 1.2 µM in the presence of zinc ion and thus the inhibitory activity was remarkably enhanced. Furthermore, in the case of Compound D, the enzyme activity was inhibited by only 13% at 100 µM in the absence of zinc ion, but IC₅₀ was 0.35 µM in the presence of zinc ion and thus the inhibitory activity was remarkably enhanced.

From these experimental results, it was obvious that the caspase 3 inhibitory action of Compounds A to D was remarkably enhanced by the presence of zinc ion.

### 5) Screening results of a known caspase inhibitor, z-VAD.FMK

A caspase 3 inhibitory action of z-VAD.FMK was examined at various concentrations (0.1, 0.3, 1, 3, 10 µM) according to the above methods 1) and 2). However, the experiment was carried out under the condition that the reaction time was 30 minutes. From the calculation of IC₅₀ under each condition of 1) and 2), IC₅₀ in the absence of zinc ion was 0.51 µM and IC₅₀ in the presence of zinc ion was 0.55 µM. Thus, there was no substantial change, and therefore, no remarkable enhancing effect of zinc ion on the caspase 3 inhibitory action of z-VAD.FMK was observed.

### Example 4

### 1) Examination of caspase 3 inhibitory mode in the case of Compound A

The caspase 3 inhibitory mode of Compound A in the presence of zinc was examined according to the methods 1) and 2) of Example 3. Compound A was mixed with the enzyme solution at concentrations of 6, 20, 60, 100, and 200 µM, and solutions of 6, 10, 20, 60, and 200 µM were used as the substrate solutions. The reaction time was 20 minutes.
The inhibitory mode was analyzed with Lineweaver-Burk plots (double reciprocal plots). According to the above method, it was found that the caspase 3 inhibitory mode of Compound A was substrate-competitive and reversible.

### 2) Examination of caspase 3 inhibitory mode in the case of Compound B

The caspase 3 inhibitory mode of Compound B in the presence of zinc was examined according to the methods 1) and 2) of Example 3. Compound B was mixed with the enzyme solution at concentrations of 20, 40, 60, 80, and 100 µM, and solutions of 6, 10, 20, and 60 µM were used as the substrate solutions. The reaction time was 10 minutes.
The inhibitory mode was analyzed with Lineweaver-Burk plots (double reciprocal plots). By the above method, it was found that the caspase 3 inhibitory mode of Compound B was substrate-competitive and reversible.

### 3) Examination of caspase 3 inhibitory mode in the case of Compound C

The caspase 3 inhibitory mode of Compound C in the presence of zinc was examined according to the methods 1) and 2) of Example 3. However, Compound C was mixed with the enzyme solution at concentrations of 1, 2, 3, 4, and 5 µM, and solutions of 6, 10, 20, 60, and 100 µM was used as the substrate solutions. The reaction time was 2 minutes.
The inhibitory mode was analyzed with Lineweaver-Burk plots (double reciprocal plots). By the above method, it was found that the caspase 3 inhibitory mode of Compound C was substrate-competitive and reversible.

As described above, the compounds found out by the screening method of the invention inhibit caspase 3 in a substrate-competitive inhibitory mode. Moreover, they inhibit caspase 3 reversibly unlike z-VAD.FMK which reacts with the enzyme.

### Example 5

### Apoptosis inhibitory action: Action of suppressing DNA fragmentation induced by Etoposide in rat hippocampal neuron

### 1) Method

Cultured rat hippocampal neuron was prepared according to a usual method (Neurochem. Int., 31(5), 715-722, 1997). Brain was taken out of a fetus of rat at day 19 of gestation, and hippocampus was picked up under a stereomicroscope. The hippocampus was treated with an enzyme, dispersed by pipetting several times, and cell clusters were removed through a filter to prepare a cell suspension. The cell was cultured at a concentration of 1.2 x 10⁵ cells/cm² in a 12-well plate coated with polylysine. A "culture medium for neuron" manufactured by Sumitomo Bakelite Co., Ltd. was used as the culture liquid. The neuronal cell at 13 to 16 days of culture was used. Etoposide was employed for inducing apoptosis of the neuronal cell. The induction of DNA fragments was evaluated as a marker of apoptosis.

Cell stimulation was carried out by replacing the cell culture medium with a serum-free medium containing 3 µg/mL Etoposide. After 6 hours from the start of the stimulation, the cell was recovered. TaKaRa ApopLadder Ex kit was used for the extraction of fragmented DNA. The fragmented DNA were subjected to electrophoresis followed by staining with CYBR Green, and the fluorescence intensity of the DNA was measured by means of a scanner manufactured by Molecular Dynamics Co.

### 2) Evaluation results of Compound C

Using the above method 1), apoptosis inhibitory action was examined by adding Compound C at various concentrations (10, 25, 50 µM) together with Etoposide. As a result, IC₅₀ of Compound C was found to be 17 µM.

### 3) Evaluation results of Compound D

In the above method 1), apoptosis inhibitory action was examined by adding Compound D together with Etoposide. As a result, Compound D exhibited a strong apoptosis inhibitory action at a concentration of 33.3 µM.

In this experimental system, no zinc is added to the cell culture medium. While in the assay of enzyme inhibition, both Compounds C and D having different skeletal structures each other showed no substantial caspase 3 inhibitory activity in the absence of zinc and remarkable inhibitory activity in the presence of zinc, both of them exerted apoptosis inhibitory action. These data demonstrate that the enzyme inhibitory activity is manifested through the intervention of zinc ion in the cell.

## Claims

1. A screening method for a cysteine protease inhibitor of which cysteine protease inhibitory activity is enhanced in the presence of endogenous polyvalent metal ion, which comprises treating a cysteine protease with a test compound in the presence of the polyvalent metal ion.

2. The screening method according to claim 1, wherein the cysteine protease inhibitor is a cystein protease inhibitor which substantially inhibits the cysteine protease in the presence of endogenous polyvalent metal ion.

3. The screening method according to claim 1, wherein the cysteine protease is caspase 3.

4. The screening method according to claim 1 or 2, wherein the polyvalent metal ion is divalent zinc ion.

5. A pharmaceutical composition which contains a cysteine protease inhibitor found out in the screening method according to claim 1.

6. The pharmaceutical composition for inhibiting apoptosis, which contains as the active ingredient a cysteine protease inhibitor of which cysteine protease inhibitory activity is enhanced in the presence of endogenous polyvalent metal ion.

7. The pharmaceutical composition according to claim 6, wherein the cysteine protease inhibitor is a cysteine protease inhibitor which substantially inhibits the cysteine protease in the presence of endogenous polyvalent metal ion.

8. The pharmaceutical composition according to claim 6 or 7, wherein the cysteine protease inhibitor is a caspase 3 inhibitor.

9. The pharmaceutical composition according to claim 6 or 7, wherein the polyvalent metal ion is divalent zinc ion.

10. The pharmaceutical composition according to claim 6 or 7, wherein the inhibition of apoptosis is the inhibition of apoptosis of a neuronal cell.

11. The pharmaceutical composition according to claim 6 or 7, wherein the inhibition of apoptosis is the inhibition of apoptosis of a hippocampal neuron.

12. Use of a cysteine protease inhibitor of which cysteine protease inhibitory activity is enhanced under the presence of endogenous polyvalent metal ion .

13. A method for inhibiting apoptosis, which comprises administering, to a patient, an effective amount of a cysteine protease inhibitor of which cysteine protease inhibitory activity is enhanced in the presence of endogenous polyvalent metal ion.

14. 1,5'-Dipropylspiro[1,8-naphthyridine-3(2H),2'-[1,3]oxathiolo[4,5-c][1,8]naphthyridine]-2,4,4'(1H,5'H)-trione, 1,5'-dipropylspiro[1,3]oxathiolo[4,5-c]quinoline-2,3'(2H)-quinoline]-2,4,4'(1H,5'H)-trione, or a pharmaceutically acceptable salt thereof.

15. A pharmaceutical composition containing 1,5'-dipropylspiro[1,8-naphthyridine-3(2H),2'-[1,3]oxathiolo[4,5-c][1,8]naphthyridine]-2,4,4'(1H,5'H)-trione, 1,5'-dipropylspiro[1,3]oxathiolo[4,5-c]quinoline-2,3'(2H)-quinoline]-2,4,4'(1H,5'H)-trione, or a pharmaceutically acceptable salt thereof.
